# EUROPEAN PATENT SPECIFICATION

(11) **EP 4 030 624 B1**
(45) Date of publication and mention of the grant of the patent: **19.06.2024**
(21) Application number: 21214711.0
(22) Date of filing: 15.12.2021
(51) Int. Cl.: H03K 17/96, A61L 2/14

(54) **TOUCH BUTTON**
BERÜHRUNGSKNOPF
BOUTON TACTILE

(30) Priority: 14.01.2021 PL 43666521
(43) Date of publication of application: 20.07.2022
(73) Proprietor: SIEC BADAWCZA LUKASIEWICZ - INSTYTUT MIKROELEKTRONIKI I FOTONIKI, 02-668 Warszawa (PL)
(72) Inventor: Zaraska, Krzysztof, 30-009 Krakow (PL)
(74) Representative: AOMB Polska Sp. z.o.o.

(56) References cited:
- EP-A2- 3 581 209
- US-A1- 2013 064 726
- US-A1- 2020 246 496

## Description

An object of the invention is a touch button, more specifically an electric diagram of a touch button that is enhanced by the possibility of self-disinfection of the button surface. Another object of the invention is a keypad circuit, having buttons according to the invention, and a device equipped with such buttons or keypad circuit. Moreover, an object of the invention is a method of operation of the device equipped with the button according to the invention. All of the above-mentioned aspects of the invention are particularly useful in the context of terminal devices accessible to the public/to large groups of people, such as self-service cash registers, intercoms, ATMs, information terminals, payment terminals, ticket machines, etc., which should be frequently disinfected so as not to pose unnecessary risks associated with potential contribution to the spread of diseases. In order to absolutely minimise the risk of spreading diseases with the contribution of these types of devices, it would be necessary to disinfect their surfaces/buttons after each use (after each user has finished using such a device), which is practically infeasible on a larger scale.

Various methods of disinfecting touch keypads are used, such as chemical disinfection, UV-C radiation or ozonisation.

A promising method for surface disinfection is the use of a cold-plasma generator which operates on the principle of barrier discharge.

From patent US 8557187, a method of sterilising a surface with plasma at atmospheric pressure generated using a surface barrier discharge is known. The system described consists of a high voltage electrode coated with a dielectric material, an electrically conductive grounded contact electrode, a high voltage source, a gas source and a gas nozzle which is placed in the immediate vicinity of the grounded contact electrode.

From the patent US 9414478, a plasma generating system is known in which an energy generating circuit is connected to electrodes, to generate a field between the electrodes so as to initiate a plasma discharge in the gap between the electrodes.

From the patent US 9572241, a system of multiple plasma emitters placed on a dielectric substrate is known. Each of the plasma emitters has a control electrode on one side of the substrate and an grounding electrode on the opposite side of the substrate. A conductive power-supply path is connected to each control electrode and a conductive grounding path is connected to each grounding electrode, wherein gas flow between the control and grounding sides takes place through holes in the substrate.

From the patent US 9737091, a sterilisation film configured to generate plasma under atmospheric pressure is known. The film comprises a flexible dielectric barrier film, an upper and a lower electrode layer that are respectively positioned on the upper and lower surfaces of the dielectric barrier film, and a lower protective layer that surrounds the exposed surface of the lower electrode layer and is formed of a dielectric material. The electrodes are electrically connected to an external power source through suitable pads.

The patent US 9757487 describes a method and a system for self-sterilisation of surfaces, in which the active and grounding electrodes are made in the form of two intersecting grids separated by a dielectric layer.

In Li et. al. article, Cold Atmospheric Plasma for Surface Disinfection, Plasma Process. Polym. 2012, 9, 585-589, a method of implementation of a self-disinfecting surface in the form of two planar comb electrodes coated with a dielectric layer is described. The cold plasma is ignited above the dielectric surface as a result of applying a high voltage to the electrodes. According to the article, turning the device on for 15 seconds is enough to reduce the number of micro-organisms on a surface by 100,000 times.

From the US patent application US2017136252, a cold plasma generator for sterilisation of moist surfaces is known, wherein this application presents the structure of such a generator in more detail, with an emphasis on the geometric arrangement of its parts and the types of materials used.

In turn, from the US patent application US2020246496, structures of plasma generators that are flexible are known.

Also, from US2013064726A1, there is known a device for sterilization/disinfection of a contaminated surface, that comprises a source of high-voltage, for the generation of non-thermal plasma, and spaced apart electrodes, that facilitate the generation of the plasma. This document however does not disclose any structure of a touch button, and does not teach of possibilities of using one of its' electrodes (used for generating the plasma) for sensing of the finger of the user (touching). This document also does not disclose the options for using a transistor, to conveniently switch the mode of operation of one of the electrodes from sensing to sterilizing.

However, state-of-the-art documents show that there are no devices in the form of touch buttons currently available that would also have the ability to quickly, automatically and highly effectively sterilise their surfaces, without user intervention and without the use of disinfectants.

An aim of the invention is therefore to provide a touch button, with an electrical circuit modified in such a way that, in addition to the typical keypad function, it enables it to be self-disinfecting. Another aim of the invention is to provide a touch keypad having several such buttons connected in an efficient manner, as well as to provide devices equipped with buttons/keypads according to the invention. An aim of the invention is also to provide a method of operation of a device equipped with the button according to the invention.

An object of the invention is a touch button comprising a first electrode, a second electrode, a readout circuit connected to the second electrode, and a grounding,
wherein the second electrode is a sensing electrode of the touch button, and the readout circuit is configured for detecting changes in the capacitance of the second electrode,
wherein the touch button also comprises a high voltage generator and a transistor,
with the high voltage output of the high voltage generator being connected to the first electrode and the chassis ground output of the high voltage generator being connected to the grounding,
where the high voltage generator is adapted to produce cold plasma on the surface of the touch button above the first and second electrodes, and
where the transistor connected between the second electrode and the grounding is configured and adapted to short-circuit the second electrode to the grounding when the high voltage generator is turned on, and
to open-circuit the connection between the second electrode and the grounding when the high voltage generator is turned off.

Preferably, it comprises an additional circuit for electrical separation of the high voltage output of the high voltage generator from the grounding.

Preferably, the said additional circuit for electrical separation is a resistor connected between the output of the high voltage generator and the grounding.

Preferably, the said additional circuit for electrical separation is an additional electrode electrically separated by a gap from the first electrode and permanently connected to the grounding, and electrically isolated from the second electrode, as well as electrically isolated from the input of the readout circuit.

Preferably, the said additional circuit for electrical separation is a means for short-circuiting the high voltage output to the grounding when the high voltage generator is turned off, preferably in the form of a switch, a relay or at least one transistor.

Preferably, the resistor is in the form of a resistive voltage divider having a longer part with higher resistance and a shorter part with lower resistance,
wherein the transistor is a field-effect transistor the gate of which is connected to the resistive voltage divider between the longer part and the shorter part of the resistive voltage divider, via a rectifier.

Preferably, the rectifier is in the form of a half-wave rectifier,
this half-wave rectifier consisting of a diode and a capacitor connected to the grounding and loaded with a discharge resistor.

Preferably, the rectifier is a Graetz bridge.

Preferably, the first electrode is connected to the output of the high voltage generator via an additional resistor.

Preferably, the first electrode consists of several active electrodes, each active electrode being connected via a fuse.

Preferably, the fuse is in the form of a resistor with a constriction in the centre or a discrete element.

Preferably, the field-effect transistor is either a MOSFET transistor or an insulated-gate bipolar transistor, IGBT, or a bipolar transistor, or a relay.

An object of the invention is also a keypad circuit with touch buttons, wherein the keypad circuit has two or more touch buttons according to the invention.

An object of the invention is also a device equipped with at least one touch button of the invention or with a keypad circuit according to the invention.

An object of the invention is also a method for controlling the device according to the invention, wherein at least one touch button, configured to operate in at least two modes, the two modes being a readout mode and a sterilisation mode, is controlled so that
in the readout mode, the high voltage generator is kept turned off and the readout circuit is powered,
in the sterilisation mode, the high voltage generator is excited and a voltage is applied to the transistor, thus short-circuiting the second electrode to the grounding,
wherein the transition between one mode and the other is based on the occurrence of predetermined circumstances, preferably detected completed operation of a particular user, or the time elapsed since the last detected interaction of a user of the device with the device and the time for which the touch button has operated in the sterilisation mode.

In the physical implementation of the button according to the invention, air is subjected to an electric field exceeding the breakdown voltage (3kV/mm), but there is a dielectric layer on the first electrode (high voltage electrode) which prevents the arc from being ignited. The electric field, however, ionises the air and produces reactive oxygen species, in particular atomic oxygen and ozone which have the ability to deactivate micro-organisms.

The proposed solution takes advantage of the fact that the cross-section of the self-disinfecting surface structure uses a standard implementation of a capacitive touch keypad. In both cases, we are dealing with an array of electrodes made on a substrate and covered with a dielectric layer. The only difference is that with plasma generation it is necessary to use two sets of electrodes with alternating polarity or hot and grounded electrodes, alternately, while with the touch keypad button - a typical one, we are dealing with only one set of electrodes for a given keypad area (button).

The use of the same electrode array for touch detection (keypad readout) and disinfection (cold plasma generation) proposed in the solution according to the invention significantly simplifies both the construction and operation of the device. Switching between keypad mode and disinfection mode is done electrically, without the need for moving parts. Disinfection can be triggered automatically, immediately after the end of service of a given user (e.g. at an ATM or payment terminal), or after a certain period of time has elapsed since detecting the last user interaction. The system does not require any chemicals or other consumables. Notification to the user that disinfection is in progress and the surface should not be touched can be done by visual and audible signalling. Because the high voltage electrode is covered with an insulator layer, touching the surface during plasma generation does not carry the risk of shocking the user unless the dielectric layer is damaged.

It is therefore important to ensure operational safety and reliability in the event of damage or intentional breakdown of the insulator layer located on the high voltage electrode. In such a situation it must be ensured above all that the current leakage through the user does not exceed the permissible safe value (20mA) and that, in the event of insulation damage (leading to arc ignition between adjacent electrodes), the damaged section is automatically and permanently disconnected.

The invention will be further explained by means of exemplary embodiments and with reference to the accompanying drawings, in which
Fig. 1 schematically shows the most basic electrical diagram of a touch button according to the invention, in its most simplified and working version,
Fig. 2 schematically shows an electrical diagram of a button having a circuit for electrical separation of the high voltage output of the high voltage generator from the grounding in the form of an additional electrode,
Fig. 3 schematically shows an electrical diagram of a button having a circuit for electrical separation of the high voltage output of the high voltage generator from the grounding in the form of a resistor,
Fig. 4 schematically shows an electrical diagram of a button having a resistor in the form of a resistive voltage divider and, a rectifier connected to the transistor,

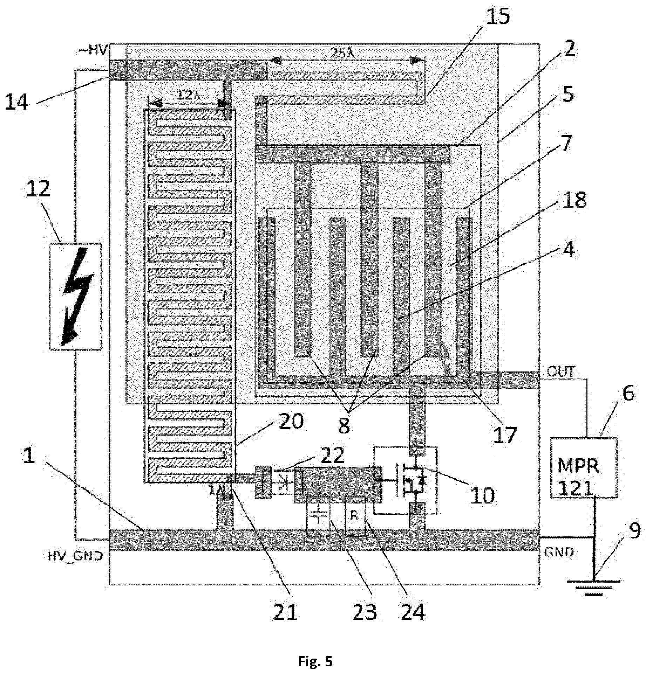

Fig. 5 schematically shows an electrical diagram of the button from Fig. 4, which is further enhanced by an additional resistor connected to the first electrode,
Fig. 6 schematically shows an electrical diagram of the button from Fig. 5 the first electrode of which is further enhanced by the use of fuses,
Fig. 7 schematically shows an electrical diagram of two touch buttons according to the invention, constituting, e.g., a two-button touch keypad,
Fig. 8 shows an exemplary implementation of a circuit for a single button with two interlocking comb electrodes underneath, and
Fig. 9. shows an example cross-section of the keypad electrode array for this button.

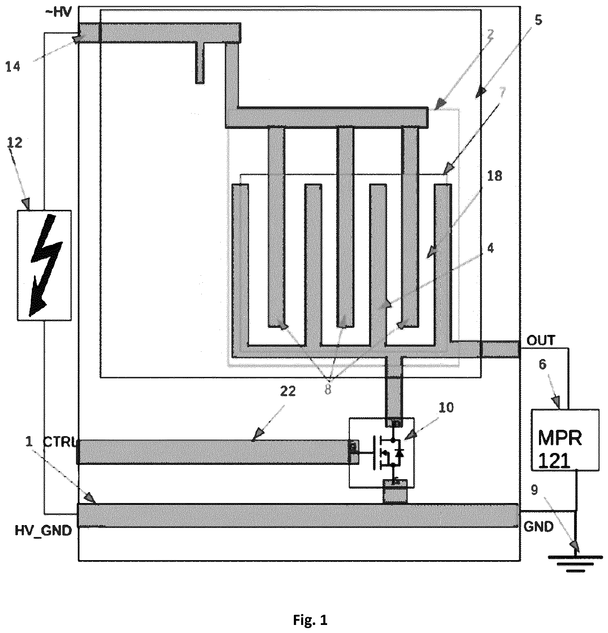

The touch button according to the invention and in its simplest form, shown in Fig. 1, has two electrodes, a first electrode 8 and a second electrode 4 which are preferably in the form of comb electrodes that interlock with each other and are placed on a substrate 1.

The first electrode 8 is electrically connected to a high voltage output 14 of a high voltage generator 12, this generator 12 also being connected to an grounding 9.

The second electrode 4 is electrically connected to a readout circuit 6 and through it to the grounding 9. The readout circuit 6 is configured so that, based on detecting a change in the capacitance of the second electrode 4, it is able to detect the proximity of the user's finger and further transmit this information, e.g., to a device equipped with such a button.

The second electrode 4 is also electrically connected to a transistor 10 that is configured to be able to short-circuit the second electrode to the grounding 9 when the high voltage generator 12 is operating and high electrical potentials may be present on the two electrodes 4, 8. By short-circuiting the second electrode 4 to the grounding 9, the readout circuit 6 is protected against the said high potentials.

In this configuration, it is possible to operate the touch button in two modes:
- readout mode, when the high voltage generator 12 is turned off, the transistor 10 does not short circuit the second electrode 4 to the chassis ground and the readout circuit 6 is able to detect changes in capacitance through the second electrode,
- sterilisation mode, when the transistor 10 turns on and short circuits the second electrode 4 to the grounding 9, while the high voltage generator 12 is turned on and causes a surface discharge on the surface of the touch button, thus sterilising the surface of the button with cold plasma.

With such a simplified form of the button according to the invention, it is important to adequately control the operation of the transistor 10 (e.g. by means of the CTRL signal) so that it shortcircuits the second electrode 4 to the grounding 9 sufficiently early when the generator 12 turns on and before dangerous potentials that could damage the readout circuit 6 would occur, as well as to terminate this short-circuit sufficiently late when the generator 12 turns off.

The touch button constructed according to the above instructions is capable of operating both as a cold plasma generator (plasma generated on the surface of the button), where the second electrode 4 is used as an grounding electrode (referring to known cold plasma generator structures), and of detecting user touch, using the second electrode 4 as a readout electrode (referring to known touch button structures), with the first electrode being in a way "idle" (due to the generator 12 being turned off and not otherwise connected to the grounding 9). With this configuration, the readout circuit 6 has a "harder" time correctly reading the proximity of a user's finger than in the case of touch buttons that have two electrodes operating in the readout mode (called readout and grounding electrodes), but by all means with the right configuration/calibration, a working button is achieved.

In a slightly more elaborate embodiment, the above-described touch button is further equipped with elements forming a circuit for electrical separation of the high voltage output 14 of the high voltage generator 12. The role of this circuit for electrical separation is to provide an additional electrode in the button readout mode, which in turn simplifies the operation of the readout circuit.

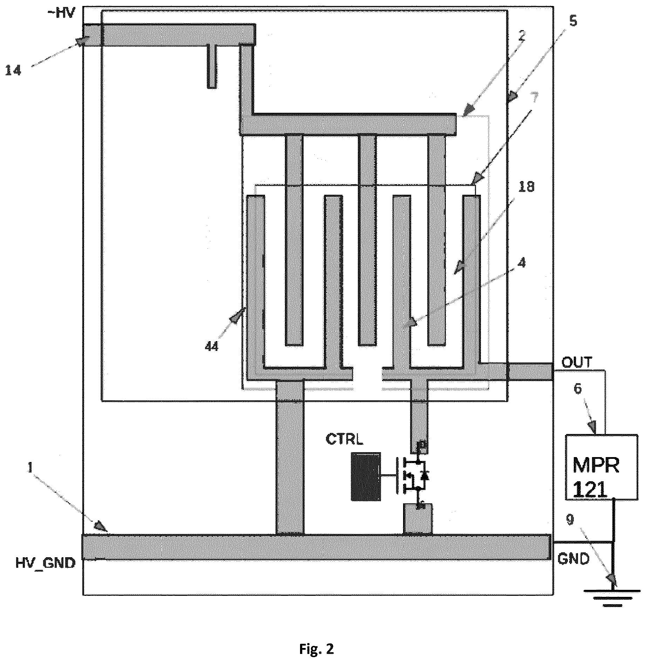

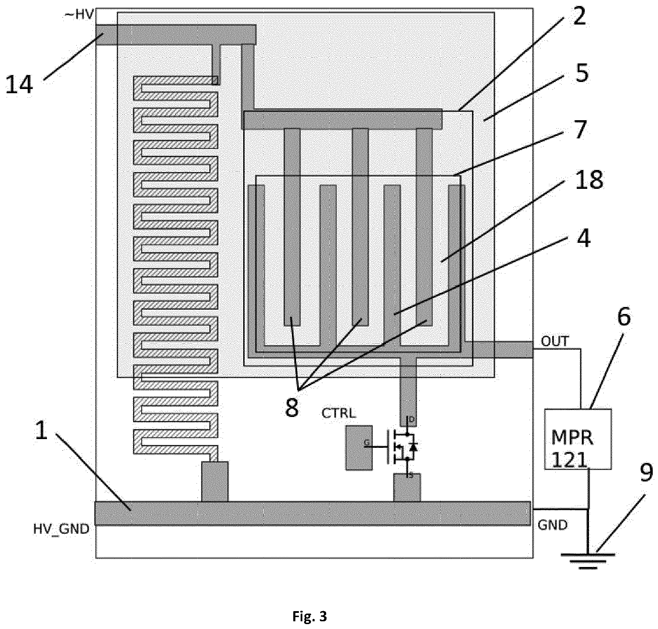

This electrical separation can be achieved in several ways:
- this may be an "ideal" separation, through the introduction of an additional element in the form of an additional electrode 44 that is electrically separated from the first electrode 8 and the second electrode 4 (e.g. made to be offset from the other two electrodes 4,8, cf. Fig. 2), yet permanently connected to the grounding 9,
- this can be a "partial" separation, through the introduction of a resistor 20 between the high voltage output 14 and the grounding 9 (cf. Fig. 3), the said resistor being characterise by a high electrical resistance (of the order of several hundred MΩ),
- this may be a "momentary" separation, through the use of means for short-circuiting and open-circuiting the high voltage output 14 and the grounding 9, for example and preferably in the form of a high voltage transistor, a switch or a relay.

If an "ideal" separation is implemented, the additional electrode can be made relatively easily, e.g. by cutting off a part of the second electrode 4 and connecting the resulting section to the grounding 9. Then, the additional electrode 44 thus created directly acts as an grounding electrode in the button readout mode.

In the case of "momentary" separation and the use of, e.g., transistors, switches or relays, it is necessary to provide additional means for controlling the operation of these elements. Although this type of modification of the button according to the invention is somewhat more expensive than, e.g., attaching/making a resistor 20, it may prove to be more preferable in some applications, due to the possibility of actively controlling/checking more of the electrical circuit elements of the button.

In case of "partial" separation, through the use of the resistor 20, on one hand, there is no risk of losing the possibility to sterilise the surface of the button (due to high resistance of the resistor 20, the current that will flow during operation of the generator 12 will be negligible from the point of view of the button), and on the other hand, the first electrode 8 can be used as an grounding electrode in the readout mode. An additional advantage of the resistor 20 is that it allows for the discharge of any voltage that may remain even after the generator 12 is turned off (e.g., in output capacitors that are typically found in high voltage generators).

Such resistor 20 may be in the form of a typical, known resistor, connected to the electrical circuit of the button, or may be made during the manufacture of the electrical circuit of the button, by making a film "meander" of a material having a high resistance per square (cf. Fig. 3).

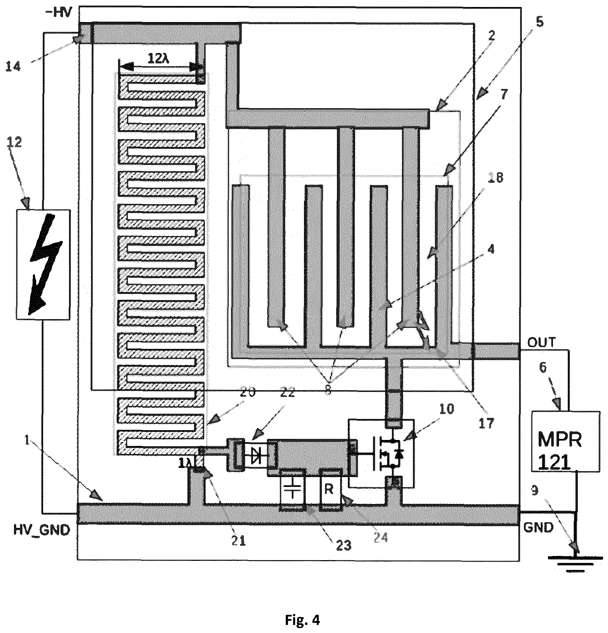

The use of the resistor 20 makes it particularly easy to make further modifications to the button according to the invention, by providing a more passive operation of the button. Fig. 4 shows a modification in which the resistor 20 is in the form of a resistive voltage divider, with a part having a higher resistance 20 (when made as a "meander" - of the longer part) and a lower resistance 21 (the shorter part of the resistor, relative to the "meandering" part). This in turn allows the voltage of the generator 12 on the longer part of the resistive voltage divider 20 to be reduced to a level that can control the transistor 10. However, to ensure that the transistor 10 is powered with a stable voltage, a voltage rectifier is inserted between the transistor 10 and the voltage divider (between its longer and shorter parts).

The button thus designed operates partially automatically - when the high voltage generator 12 is turned on, the transistor 10 will be powered simultaneously to the voltage appearing on the first electrode in order to short-circuit the second electrode 4 to the grounding 9. Also - when the generator 12 is turned off, the charges remaining on the first electrode 8 or in the generator 12 will be discharged through the voltage divider 20, 21, which in turn will also cause the transistor 10 to be turned off and the second electrode 4 to be disconnected from the grounding 9, allowing the button to enter the readout mode. It can be seen that with this configuration it is no longer necessary to control the operation of the transistor 10 by additional control signals (in Fig. 4 the CTRL marking is no longer present), which is preferable in view of the simplicity of the construction and operation of the button according to the invention.

The above-described button according to the invention, in any of its configurations, can be equipped with additional protective means that would protect users from electric shock in a situation where the button is partially damaged (e.g. the dielectric layer over the first electrode 8 is damaged). In case of such a situation, it is preferable to provide an additional resistor 15, placed between the high voltage output 14 of the generator 12 and the first electrode 8.

Preferably, the additional resistor 15 is characterised by a high electrical resistance (of the order of several hundred kΩ), selected to reduce the current that could flow through this additional resistor 15 to safe values during the operation of the high voltage generator 12. Such values are usually safely around 20 mA. The additional resistor 15 may be a standard resistor, or may be manufactured as a path from a high electrical resistance material, in a manner analogous to the previously described resistor 20.

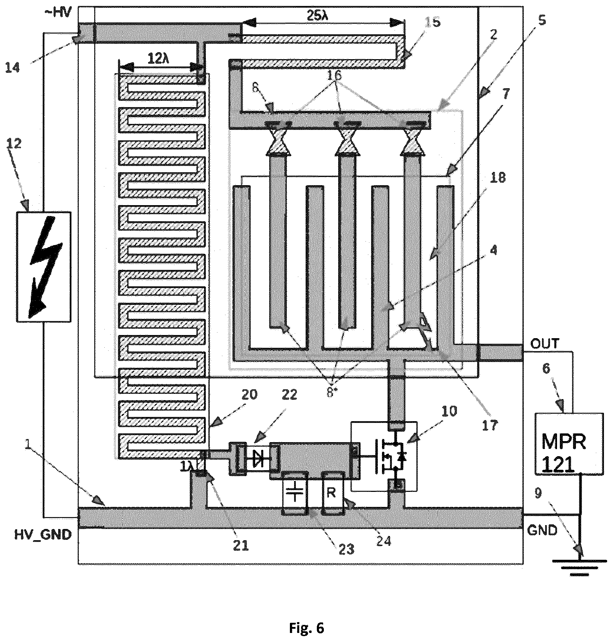

Another way to protect the users and the structure itself of the touch button from overvoltage/electric shock may be to use the first electrode 8 consisting of several independent active electrodes 8* (where one active electrode 8* is one "branch/teeth" of the first electrode 8, cf. Fig. 6) and to connect each of them via a fuse 16. In such a configuration, if an electrical insulation breakdown occurs and an electric arc occurs (whether between the electrodes or between one of the active electrodes 8* and the user's finger), the fuse 16, when tripped, will disconnect the respective one(s) of the active electrodes 8*, excluding any further possibility of an electric arc occurring but retaining the ability (somewhat deteriorated due to the loss of one or more of the active electrodes 8*) of the touch button to operate. This type of modification may be provided with respect to any form of the touch button according to the invention.

The fuse(s) 16 may be in the form of e.g. classic fuses in the form of discrete elements, or may be in the form of a flat resistor with a constriction in the centre adapted to evaporate in the event of electric arc ignition (cf. Fig. 6).

In one embodiment (also corresponding to the diagram shown in Fig. 6), the button was realised in the form of a thick-film structure on the ceramic substrate 1. The electrodes and other electrical connections within the substrate 1 were made of a conductive material, in the embodiment silver paste but it could also be gold paste or ITO. The dielectric layer covering the electrodes was in the form of a printed and burned glaze but can also be in the form of a ceramic film laminated onto the substrate 1. In another embodiment, both the substrate 1 and the dielectric were realised as two layers in the process of realising low temperature co-fired ceramics (LTCC).

In the embodiment discussed, the divider 20, 21 is made using a 1MΩ/□ high-ohm paste, where the resistance of the resistor 20 is 300MΩ and of the resistor 21 is 1MΩ. For an operating voltage of 10kV this means that a small power of 0.33W is released on the divider. The divider voltage (which is approximately 33V) is rectified by a half-wave rectifier consisting of a diode 22 and a capacitor 23 loaded with a discharge resistor 24. This voltage is then applied to the gate of the field-effect transistor 10 and causes automatic turning on at the moment the HV generator is turned on.

When the button circuit is powered with a voltage of 10kV, limiting the leakage current by the user to the permissible value of 20mA requires the use of a 500kΩ resistor. Such a resistor may be made in the form of a rectangle or meander printed with a resistive paste with a resistivity of 10 kΩ/□ and a length of 50 squares.

In the embodiment discussed, the fuse 16 is made in the form of a thick-layer resistor with a constriction so that when the arc is ignited (in case of the dielectric layer breakdown), the area of the constriction burns through, with the aim of permanently turning off the active electrode in the area where the breakdown has occurred. On the constriction that is 0.1x0.1mm in size and is made of 10 kΩ/□ resistive paste, loaded with an arc current of 20mA (resulting from limiting with the resistor 15), a power of 4W will be released which corresponds to a power density of 400W/mm2, sufficient to destroy the protective resistor 16.

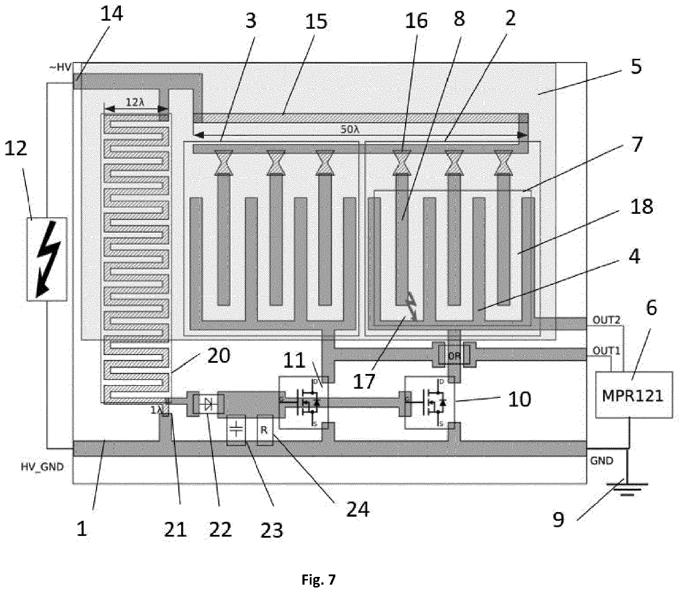

In turn, Fig. 7 shows a diagram of combining the touch buttons according to the invention into a multi-button (here, two-button) keypad. Although Fig. 7 shows a diagram of buttons with a slightly more elaborate configuration (with the voltage divider 20, 21, the additional resistor 15 and the fuses 16), it is obvious that also more basic button variants can be combined in an analogous manner. It can be seen that each button has its own first 8 and second 4 electrodes, its own transistor 10 and its own connection to the readout circuit 6 (and possibly its own fuses 16). In turn, the high voltage generator 12, as well as the optional voltage divider 20, 21, the additional resistor 15, the rectifier and the readout circuit 6 are common to two or more touch buttons.

In one embodiment, the touch button keypad circuit has two or more touch buttons according to the invention,
wherein the touch buttons have their own sets of the first electrodes 8 and the second electrodes 4 together with their corresponding fuses 16,
wherein the touch buttons are connected to the common additional resistor 15,
wherein the touch buttons have their own transistors 10, 11, connected to one common rectifier and to the common grounding 9,
wherein the touch buttons have separate connections to the readout circuit 6.

Such examples of keypads are easily configurable, taking into account only the need for appropriate adjustment of design/operating parameters which may vary due to the size of the resulting keypad, and thus the operating voltages of the generator 12 in order to sterilise the keypad at a given time, and thus the resistance/current values for tripping the resistors/fuses.

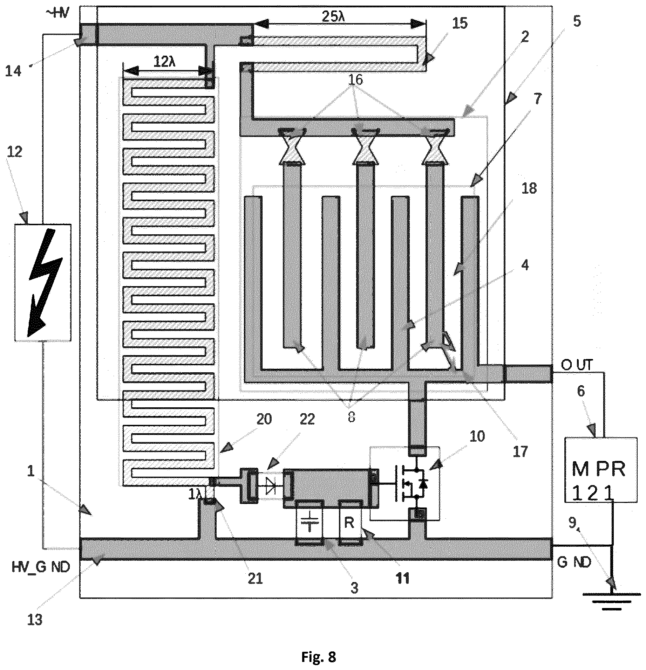

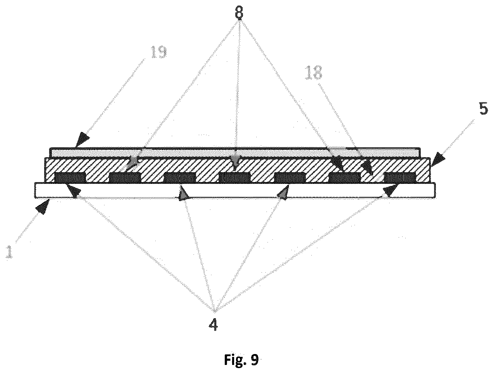

The invention, according to some preferable embodiments, will be explained in more detail using the example of the electrical circuit of the capacitive touch keypad shown in the drawings. Fig. 8 shows an implementation of the circuit for a single button under which there are two interlocking comb electrodes, and Fig. 9 of the drawings shows a cross-section of an electrode array of the keypad for this button.

The electrical circuit of the touch keypad according to the invention has a touch button equipped with a set of active readout electrodes 4, and active electrodes 8 deposited on a substrate 1, separated by a channel 18 and covered with an insulator layer 5. Each of the readout electrodes 4 is connected to a readout circuit 6. In turn, each electrode 8 is powered by an individual protective resistor 16, in the embodiment in the form of a thick-film element. The set of the active electrodes 8 is connected to an output 14 of a high voltage generator 12 via a current limiting resistor 15, in the embodiment made in the form of a thick-layer element. The output 14 of the high voltage generator is also connected to a ground 9 of the circuit via a resistor 20 that acts as a discharge resistor for output capacitors of the HV power supply 12. This connection may also be made via a resistive voltage divider 20 and 21, preferably made as a thick-film element. In turn, the readout electrodes 4 of the buttons are connected to the grounding 9 of the circuit through a field-effect transistor 10 the gate of which is connected to the output of the resistive voltage divider 20 and 21 through a rectifying element, in the embodiment through a half-wave rectifier 22 but this may also be a Graetz bridge. The field-effect transistor 10 is a MOSFET but can be an insulated-gate bipolar transistor, IGBT, or a relay.

In the example circuit, the electrodes 4 and 8 are made in the form of two interlocking comb electrodes. One of the electrodes, the active electrode 8 is used solely for plasma generation when it is connected to the high voltage source, the generator 12 in disinfection mode and disconnected in readout mode. In turn, the second electrode, the readout electrode 4 is used in the readout mode (connected to the readout circuit 6) or short-circuited to the ground 9 (grounding) in the plasma generation mode using the MOSFET type transistor 10. This solution allows the readout electrode 4 to be permanently connected to the readout circuit 6 without having to be disconnected during plasma generation and without risk of damage to the readout circuit. From the point of view of the readout circuit, an additional capacitance appears in the readout mode between the readout electrode 4 and the active electrode 8 connected to the inactive output 14 of the generator 12, but this capacitance is fixed and can be compensated for in the calibration process of the readout circuit.

The circuit was realised in the form of a thick-layer structure on the ceramic substrate 1. Embodiment limited to a single button 2 The button is made in the form of the readout electrode 4 and the comb-shaped active electrode 8 covered with the dielectric layer 5. Electrodes and other electrical connections within the substrate 1 are made of a conductive material, in the embodiment silver paste but it can also be gold paste or ITO.

The dielectric layer 5 is in the form of a printed and burned glaze but can also be in the form of a ceramic film laminated onto the substrate 1. In another embodiment, both the substrate 1 and the dielectric 5 were realised as two layers in the process of realising low temperature co-sintered ceramics (LTCC).

The readout electrode 4 is connected to the capacitance readout circuit 6, realised based on the MPR121 integrated circuit. Touching the dielectric 5 with a finger, in the area above the readout electrode 4, changes the electrical capacitance measured by the readout circuit 6.

The keypad is disinfected by igniting a cold plasma 19 over the surface of the dielectric 5 in an active area 7 due to an electric field created by applying a high voltage between the active electrodes 8 and the readout electrodes 4. In this mode of operation, the readout electrode is short-circuited to the grounding 9 by the MOSFET type field-effect transistor 10. This makes it possible simultaneously to close the high-voltage circuit and to protect the readout circuit 6, the input of which is short-circuited to the ground, so that there is no danger of its input circuits being damaged by an electrical charge from plasma generation. The active electrodes 8 are powered by the high voltage power supply 12. The HV power supply is connected between a HV ground contact 13 short-circuited to the grounding 9 and the HV input contact 14. The active electrodes 8 are powered by a protective resistor 15 the task of which is to limit the leakage current in the event of a breakdown or damage of the dielectric layer 5. When the circuit is powered with a voltage of 10kV, limiting the leakage current by the user to an acceptable value of 20mA requires a 500kΩ resistor to be used. Such a resistor may be made in the form of a rectangle or meander printed with a resistive paste with a resistivity of 10 kΩ/□ and a length of 50 squares.

The power supply to each of the active electrodes 8 is provided by the protective resistor 16. Under normal conditions of plasma generation, the circuit operates as a capacitor with the dielectric layer 5 filled in in the form of a channel 18 between the electrodes 4 and 8. In the event of a breakdown of the dielectric layer, an electric arc 17 may be ignited between the active electrode 8 and the grounded readout electrode 4.

Because the arc resistance is lower than the channel resistance of the dielectric 18, virtually all the current in the active area of the keypad 2 flows through the ignited arc 17. The value of this current results from the operating voltage of the HV generator 12 and from the value of the protective resistor 15 and, as explained above, should be limited to a maximum of 20mA for reasons of safety in use. At the same time, the entire arc current flows through the fuse 16 of the active electrode 8 in the region where the breakdown has occurred. This fuse is made in the form of a thick-layer resistor with a constriction so that when the arc is ignited, it burns through in the area of the constriction in order to permanently turn off the active electrode in the region where the breakdown has occurred. On a constriction that is 0.1x0.1mm in size and is made of 10 kΩ/□ resistive paste, loaded with an arc current of 20mA (resulting from limiting with the resistor 15, a power of 4W will be released, which corresponds to a power density of 400W/mm2, sufficient to destroy the protective resistor 16.

Switching of the circuit between operation mode and disinfection mode occurs automatically when a positive or variable high voltage is applied between the contacts ~HV 14 and HV_GND 13. The operating voltage is applied to the resistive voltage divider 20 and 21, in the embodiment with a ratio of approximately 300:1. This divider consists of a meander resistor 20 and a square resistor 21.

In the embodiment, the divider is made with 1MΩ/□ high-ohm paste, the resistance of the resistor 20 is 300MΩ and of the resistor 21 is 1MΩ. For an operating voltage of 10kV this means that a small power of 0.33W is released on the divider. The divider voltage (which is approximately 33V) is rectified by a half-wave rectifier consisting of a diode 22 and a capacitor 3 loaded with a discharge resistor 11. This voltage is then applied to the gate of the field-effect transistor 10 and causes automatic turning on at the moment the HV generator is turned on.

In the plasma generation mode, current flows in the high voltage circuit from the output 14 of the HV generator 12 through the current limiting resistor 15 and the protective resistors 16 to the active electrodes 8, then through the plasma 19 in the active area 7 in the air above the keypad to the readout electrode 4 connected to the ground 9 through the turned on transistor 10. The appearance of a high voltage at the output 14 causes a proportionally reduced voltage to appear at the output of the divider 20, 21. This voltage is rectified by the rectifier 22 and the capacitor 3, causing the transistor 10 to be turned on automatically when the HV power supply is turned on and the electrode 4 to be connected to the ground. Turning off the HV generator causes the capacitor 3 to be discharged by the resistor 11, which results in the transistor 10 being turned off, causing an automatic transition to readout mode.

Devices equipped with touch buttons/keypads with the buttons according to the invention can in particular be ATMs, infokiosks, ticketing terminals, self-service terminals at airports, payment terminals, etc. - in short, all devices which can be used by many people and which devices should be sterilised to stop the potential spread of diseases.

## Claims

1. A touch button, comprising a first electrode (8), a second electrode (4), a readout circuit (6) connected to the second electrode (4), and an grounding (9),
wherein the second electrode (4) is a sensing electrode of the touch button, and the readout circuit (6) is configured for detecting changes in the capacitance of the second electrode (4),
wherein the touch button also comprises a high voltage generator (12) and a transistor (10),
wherein a high voltage output (14) of the high voltage generator (12) is connected to the first electrode (8) and a ground output of the high voltage generator (12) is connected to the grounding,
where the high voltage generator (12) is adapted to produce cold plasma on the surface of the touch button above the first and second electrodes (8, 4), and
where the transistor (10) connected between the second electrode (4) and the grounding (9) is configured and adapted to short-circuit the second electrode (4) with the grounding (9) when the high voltage generator (12) is turned on, and
to open-circuit the connection between the second electrode (4) and the grounding (9) when the high voltage generator (12) is turned off.

2. The touch button according to claim 1, wherein it comprises an additional circuit for electrical separation of the high voltage output (14) of the high voltage generator (12) from the grounding (9).

3. The touch button according to claim 2, wherein the said additional circuit for electrical separation is a resistor (20) connected between the output (14) of the high voltage generator (12) and the grounding (9).

4. The touch button according to claim 2, wherein the said additional circuit for electrical separation is constituted by an additional electrode (44), electrically separated by a gap (18) from the first electrode (8) and permanently connected to the grounding (9), and electrically isolated from the second electrode (4), as well as electrically isolated from the input of the readout circuit (6).

5. The touch button according to claim 2, wherein the said additional circuit for electrical separation is constituted by a means for short-circuiting the high voltage output (14) to the grounding (9) when the high voltage generator (12) is turned off, preferably in the form of a switch, a relay or at least one transistor.

6. The touch button according to claim 3, **wherein** the resistor (20) is in the form of a resistive voltage divider (20, 21) having a longer part with a higher resistance and a shorter part with a lower resistance, wherein the transistor (10) is a field-effect transistor the gate of which is connected to a resistive voltage divider between the longer part (20) and the shorter part (21) of the resistive voltage divider (20, 21), via a rectifier.

7. The touch button according to claim 6, **wherein** the rectifier is in the form of a half-wave rectifier, where this half-wave rectifier consists of a diode (22) and a capacitor (23) connected to the grounding (9) and loaded with a discharge resistor (24).

8. The touch button according to claim 6, **wherein** the rectifier is a Graetz bridge.

9. The touch button according to any of claims 1 to 8, **wherein** the first electrode (8) is connected to the output (14) of the high voltage generator (12) via an additional resistor (15).

10. The touch button according to any of claims 1 to 9, **wherein** the first electrode (8) consists of several active electrodes (8*), each active electrode (8*) being connected via a fuse (16).

11. The touch button according to claim 10, **wherein** the fuse (16) is in the form of a resistor with a constriction in the centre or a discrete element.

12. The touch button according to claim 6, 7, 8, 9, 10 or 11, **wherein** the field-effect transistor (10) is either a MOSFET transistor or an insulated gate bipolar transistor, IGBT, or a relay.

13. A keypad circuit with touch buttons, wherein the keypad circuit has two or more touch buttons as defined according to any of claims 1 to 12.

14. A device equipped with at least one touch button according to any of the preceding claims 1 to 12, or with the keypad circuit according to claim 13.

15. A method for controlling a device as defined in claim 14, wherein at least one touch button, configured to operate in at least two modes, the two modes being a readout mode and a sterilisation mode, is controlled so that
in the readout mode, the high voltage generator (12) is kept turned off and the readout circuit is powered,
in the sterilisation mode, the high voltage generator (12) is excited and a voltage is applied to the transistor (10), thus short-circuiting the second electrode (4) to the grounding (9),
wherein the transition between one mode and the other is based on the occurrence of predetermined circumstances, preferably detected completed operation of a particular user, or the time elapsed since the last detected interaction of a user of the device with the device and the time for which the touch button has operated in the sterilisation mode.

## Patentansprüche

1. Berührungstaste, die Folgendes umfasst: eine erste Elektrode (8), eine zweite Elektrode (4), eine Ausleseschaltung (6), die mit der zweiten Elektrode (4) verbunden ist, und eine Erdung (9),
wobei die zweite Elektrode (4) eine Fühlerelektrode der Berührungstaste ist und die Ausleseschaltung (6) zum Erfassen von Änderungen der Kapazität der zweiten Elektrode (4) konfiguriert ist,
**wobei** die Berührungstaste auch Folgendes umfasst
einen Hochspannungsgenerator (12) und einen Transistor (10),
wobei ein Hochspannungsausgang (14) des Hochspannungsgenerators (12) mit der ersten Elektrode (8) verbunden ist und ein Erdeausgang des Hochspannungsgenerators (12) mit der Erdung verbunden ist,
wobei der Hochspannungsgenerator (12) geeignet ist, kaltes Plasma auf der Oberfläche der Berührungstaste oberhalb der ersten und zweiten Elektrode (8, 4) zu erzeugen, und
wobei der Transistor (10), der zwischen die zweite Elektrode (4) und die Erdung (9) geschaltet ist, so konfiguriert und angepasst ist, dass er die zweite Elektrode (4) mit der Erdung (9) kurzschließt, wenn der Hochspannungsgenerator (12) eingeschaltet wird, und
die Verbindung zwischen der zweiten Elektrode (4) und der Erdung (9) zu unterbrechen, wenn der Hochspannungsgenerator (12) ausgeschaltet ist.

2. Die Berührungstaste nach Anspruch 1, **wobei** sie eine zusätzliche Schaltung zur elektrischen Trennung des Hochspannungsausgangs (14) des Hochspannungsgenerators (12) von der Erdung (9) aufweist.

3. Die Berührungstaste nach Anspruch 2, **wobei** die zusätzliche Schaltung zur elektrischen Trennung ein Widerstand (20) ist, der zwischen dem Ausgang (14) des Hochspannungsgenerators (12) und der Erdung (9) angeschlossen ist.

4. Die Berührungstaste nach Anspruch 2, **wobei** die zusätzliche Schaltung zur elektrischen Trennung aus einer zusätzlichen Elektrode (44) besteht, die durch einen Spalt (18) von der ersten Elektrode (8) elektrisch getrennt und ständig mit der Erdung (9) verbunden ist, und die von der zweiten Elektrode (4) elektrisch isoliert ist, sowie vom Eingang der Ausleseschaltung (6) elektrisch isoliert ist.

5. Die Berührungstaste nach Anspruch 2, **wobei** die zusätzliche Schaltung zur elektrischen Trennung durch ein Mittel zum Kurzschließen des Hochspannungsausgangs (14) mit der Erdung (9) bei abgeschaltetem Hochspannungsgenerator (12), vorzugsweise in Form eines Schalters, eines Relais oder mindestens eines Transistors, gebildet wird.

6. Die Berührungstaste nach Anspruch 3, **wobei** der Widerstand (20) die Form eines ohmschen Spannungsteilers (20, 21) hat, der einen längeren Teil mit einem höheren Widerstand und einen kürzeren Teil mit einem niedrigeren Widerstand aufweist,
wobei der Transistor (10) ein Feldeffekttransistor ist, dessen Gate über einen Gleichrichter mit einem ohmschen Spannungsteiler zwischen dem längeren Teil (20) und dem kürzeren Teil (21) des ohmschen Spannungsteilers (20, 21) verbunden ist.

7. Die Berührungstaste nach Anspruch 6, **wobei** der Gleichrichter als Einweggleichrichter ausgebildet ist,
wobei dieser Einweggleichrichter aus einer Diode (22) und einem Kondensator (23) besteht, der mit der Erdung (9) verbunden und mit einem Entladewiderstand (24) belastet ist.

8. Die Berührungstaste nach Anspruch 6, **wobei** der Gleichrichter eine Graetz-Brücke ist.

9. Die Berührungstaste nach einem der Ansprüche 1 bis 8, **wobei** die erste Elektrode (8) über einen zusätzlichen Widerstand (15) mit dem Ausgang (14) des Hochspannungsgenerators (12) verbunden ist.

10. Die Berührungstaste nach einem der Ansprüche 1 bis 9, **wobei** die erste Elektrode (8) aus mehreren aktiven Elektroden (8*) besteht, wobei jede aktive Elektrode (8*) über eine Sicherung (16) angeschlossen ist.

11. Die Berührungstaste nach Anspruch 10, **wobei** die Sicherung (16) als Widerstand mit einer Einschnürung in der Mitte oder als diskretes Element ausgebildet ist.

12. Die Berührungstaste nach Anspruch 6, 7, 8, 9, 10 oder 11, **wobei** der Feldeffekttransistor (10) entweder ein MOSFET-Transistor oder ein bipolarer Transistor mit isoliertem Gate, IGBT, oder ein Relais ist.

13. Eine Tastaturschaltung mit Berührungstasten, **wobei** die Tastaturschaltung zwei oder mehr Berührungstasten gemäß einem der Ansprüche 1 bis 12 aufweist.

14. Ein Gerät mit mindestens einer Berührungstaste nach einem der vorhergehenden Ansprüche 1 bis 12 oder mit der Tastaturschaltung nach Anspruch 13.

15. Ein Verfahren zur Steuerung eines Geräts nach Anspruch 14, **wobei** mindestens eine Berührungstaste, die so konfiguriert ist, dass sie in mindestens zwei Modi arbeitet, wobei die beiden Modi ein Auslesemodus und ein Sterilisationsmodus sind, so gesteuert wird, dass
im Auslesemodus der Hochspannungsgenerator (12) ausgeschaltet bleibt und die Ausleseschaltung mit Strom versorgt wird,
im Sterilisationsmodus der Hochspannungsgenerator (12) erregt wird und eine Spannung an den Transistor (10) angelegt wird, wodurch die zweite Elektrode (4) mit der Erdung (9) kurzgeschlossen wird,
wobei der Übergang zwischen dem einen und dem anderen Modus auf dem Auftreten vorbestimmter Umstände beruht, vorzugsweise auf einer erfassten abgeschlossenen Bedienung eines bestimmten Benutzers oder auf der Zeit, die seit der letzten erfassten Interaktion eines Benutzers des Geräts mit dem Gerät verstrichen ist, und auf der Zeit, in der die Berührungstaste im Sterilisationsmodus gearbeitet hat.

## Revendications

1. Un bouton tactile comprenant une première électrode (8), une seconde électrode (4), un circuit de lecture (6) connecté à la seconde électrode (4), et une mise à la terre (9),
dans lequel la seconde électrode (4) est une électrode de détection du bouton tactile, et le circuit de lecture (6) est configuré pour détecter les changements dans la capacité de la seconde électrode (4),
dans lequel le bouton tactile comprend également
un générateur de haute tension (12) et un transistor (10),
dans lequel une sortie haute tension (14) du générateur haute tension (12) est reliée à la première électrode (8) et une sortie de masse du générateur haute tension (12) est reliée à la mise à la terre,
où le générateur haute tension (12) est adapté pour produire un plasma froid sur la surface du bouton tactile au-dessus des première et seconde électrodes (8, 4), et
où le transistor (10) relié entre la seconde électrode (4) et la mise à la terre (9) est configuré et adapté pour court-circuiter la seconde électrode (4) avec la mise à la terre (9) lorsque le générateur haute tension (12) est mis en marche, et
pour ouvrir le circuit qui relie la seconde électrode (4) à la mise à la terre (9) lorsque le générateur haute tension (12) est éteint.

2. Le bouton tactile selon la revendication 1, dans lequel il comprend un circuit supplémentaire pour la séparation électrique de la sortie haute tension (14) du générateur haute tension (12) par rapport à la mise à la terre (9).

3. Le bouton tactile selon la revendication 2, dans lequel ledit circuit supplémentaire pour la séparation électrique est une résistance (20) reliée entre la sortie (14) du générateur haute tension (12) et la mise à la terre (9).

4. Le bouton tactile selon la revendication 2, dans lequel ledit circuit supplémentaire pour la séparation électrique est constitué d'une électrode supplémentaire (44), séparée électriquement par un espace (18) depuis la première électrode (8) et reliée en permanence à la mise à la terre (9), et isolée électriquement par rapport à la seconde électrode (4), ainsi qu'isolée électriquement par rapport à l'entrée du circuit de lecture (6).

5. Le bouton tactile selon la revendication 2, dans lequel ledit circuit supplémentaire pour la séparation électrique est constitué d'un moyen pour court-circuiter la sortie haute tension (14) à la mise à la terre (9) lorsque le générateur haute tension (12) est éteint, de préférence sous la forme d'un interrupteur, d'un relais ou d'au moins un transistor.

6. Le bouton tactile selon la revendication 3, dans lequel la résistance (20) se présente sous la forme d'un diviseur de tension résistif (20, 21) ayant une partie plus longue avec une résistance plus élevée et une partie plus courte avec une résistance plus faible,
dans lequel le transistor (10) est un transistor à effet de champ dont la grille est reliée à un diviseur de tension résistif entre la partie plus longue (20) et la partie plus courte (21) du diviseur de tension résistif (20, 21), par l'intermédiaire d'un redresseur.

7. Le bouton tactile selon la revendication 6, dans lequel le redresseur se présente sous la forme d'un redresseur demi-onde,
où ce redresseur demi-onde est constitué d'une diode (22) et d'un condensateur (23) relié à la mise à la terre (9) et chargé d'une résistance de décharge (24).

8. Le bouton tactile selon la revendication 6, dans lequel le redresseur est un pont de Graetz.

9. Le bouton tactile selon l'une des revendications 1 à 8, dans lequel la première électrode (8) est reliée à la sortie (14) du générateur haute tension (12) par l'intermédiaire d'une résistance supplémentaire (15).

10. Le bouton tactile selon l'une des revendications 1 à 9, dans lequel la première électrode (8) est constituée de plusieurs électrodes actives (8*), chaque électrode active (8*) étant reliée par l'intermédiaire d'un fusible (16).

11. Le bouton tactile selon la revendication 10, dans lequel le fusible (16) se présente sous la forme d'une résistance avec un étranglement au centre ou d'un élément discret.

12. Le bouton tactile selon la revendication 6, 7, 8, 9, 10 ou 11, dans lequel le transistor à effet de champ (10) est soit un transistor MOSFET, soit un transistor bipolaire à grille isolée (IGBT), soit un relais.

13. Un circuit de clavier avec des boutons tactiles, dans lequel le circuit de clavier a deux boutons tactiles ou plus tels que définis selon l'une des revendications 1 à 12.

14. Un dispositif équipé d'au moins un bouton tactile selon l'une quelconque des revendications précédentes 1 à 12, ou du circuit de clavier selon la revendication 13.

15. Un procédé pour contrôler un dispositif tel que défini dans la revendication 14, dans lequel au moins un bouton tactile, configuré pour fonctionner dans au moins deux modes, les deux modes étant un mode de lecture et un mode de stérilisation, est contrôlé de telle sorte que
en mode de lecture, le générateur haute tension (12) reste éteint et le circuit de lecture est alimenté,
en mode de stérilisation, le générateur haute tension (12) est excité et une tension est appliquée au transistor (10), court-circuitant ainsi la seconde électrode (4) à la mise à la terre (9),
dans lequel la transition entre un mode et l'autre est basée sur l'apparition de circonstances prédéterminées, de préférence l'opération complète détectée d'un utilisateur particulier, ou le temps écoulé depuis la dernière interaction détectée d'un utilisateur du dispositif avec le dispositif et le temps pendant lequel le bouton tactile a fonctionné en mode de stérilisation.
